Europäisches Patentamt

European Patent Office

Office européen des brevets

(19)

(11) Veröffentlichungsnummer : **0 054 654
B1**

(12) **EUROPÄISCHE PATENTSCHRIFT**

(45) Veröffentlichungstag der Patentschrift :
16.04.86

(51) Int. Cl.⁴ : **A 61 N   1/08**

(21) Anmeldenummer : **81108434.2**

(22) Anmeldetag : **16.10.81**

(54) **Elektromedizinisches Gerät.**

(30) Priorität : **18.12.80 DE 3047861**

(43) Veröffentlichungstag der Anmeldung :
**30.06.82 Patentblatt 82/26**

(45) Bekanntmachung des Hinweises auf die Patenterteilung : **16.04.86 Patentblatt 86/16**

(84) Benannte Vertragsstaaten :
**AT CH IT LI NL**

(56) Entgegenhaltungen :
**EP-A- 0 026 324
DE-A- 1 648 905
DE-A- 2 352 631
US-A- 2 843 129
US-A- 3 349 309
ELECTRICAL DESIGN NEWS, Band 25, Nr. 14, August
1980, Seite 130, Denver, U.S.A., V.L. PATIL et al.:
"Pulse-with sieve counts the good and bad"**

(73) Patentinhaber : **Siemens Aktiengesellschaft
Berlin und München Wittelsbacherplatz 2
D-8000 München 2 (DE)**

(72) Erfinder : **Szehi, Erich
Fillenbaumgasse 41
A-1210 Wien (AT)**

EP 0 054 654 B1

## Beschreibung

Die Erfindung betrifft ein Reizstromgerät gemäß Oberbegriff von Anspruch 1.

Solche Reizstromgeräte müssen mit Sicherheitsschaltungen entsprechend den VDE-Vorschriften ausgerüstet sein. Ein Gerät der eingangs genannten Art mit einer solchen Sicherheitsschaltung ist in der nur nach Art. 54 Abs. 3 zu berücksichtigenden europ. Patentschrift 0 026 324 beschrieben. Dort wird der Istwert des Patientenstromes mit einem Sollwert verglichen und bei Abweichungen eine Abschaltung des Gerätes ausgelöst.

Trotz einer solchen Schutzeinrichtung kann es jedoch zu einer Gefährdung des Patienten kommen, wenn das Reizstromgerät einen Impulsstrom abgibt, dessen Soll-Parameter, wie Impulsdauer und Impulsabstand, in einem weiten Bereich einstellbar sind. Nach physiologischen Gegebenheiten ist nämlich die notwendige Intensität des Reizstromes, die zu einer Schwellenzuckung bzw. zu einer Kontraktion des Muskels führt, von der Dauer der Einwirkung mit Reizstrom abhängig. Um mit einer kurzen Impulsdauer der Größenordnung von ungefähr 30 ms Schwellenzuckungen zu erreichen, sind erhebliche Stromstärken erforderlich. Dies ist dann ungefährlich, wenn es sich nur um kurzzeitige Impulse handelt. Würde jedoch im Grenzfall eines Gleichstromes die gleiche Stromstärke und damit eine entsprechend hohe Betriebsspannung dem Patienten zugeführt, so könnte die insgesamt verabreichte Strommenge zu einer Gefährdung des Patienten führen. Eine ganz entsprechende Wirkung hätte ebenso die Verkleinerung der Impulspausen, die z. B. ebenfalls im Grenzfall zur Abgabe von Gleichstrom mit unerwünscht hoher Strommenge führen würde.

Bei solchen Reizstromgeräten, die mit Impulsströmen arbeiten, ist die beim Stand der Technik vorbekannte Überwachung der Stromstärke noch nicht hinreichend. Insbesondere bei Ausfall eines Bauelementes kann es bei einem Gerät der eingangs genannten Art zu einer Impulsverdichtung kommen, die den Patienten gefährden kann, aber keine Abschaltung des Gerätes auslöst.

Aufgabe der Erfindung ist es daher, ein Reizstromgerät zu schaffen, das auch bei Anwendung von Impulsströmen keine Überbelastung des Patienten zuläßt.

Die Lösung dieser Aufgabe ist in Anspruch 1 gekennzeichnet.

Weitere Einzelheiten und Vorteile der Erfindung werden anhand des in den Figuren dargestellten Ausführungsbeispiels näher erläutert. Es zeigen

Figur 1 das Blockschaltbild eines Reizstromgerätes mit einer Sicherheitsschaltung,

Figur 2 Einzelheiten der Sicherheitsschaltung von Fig. 1.

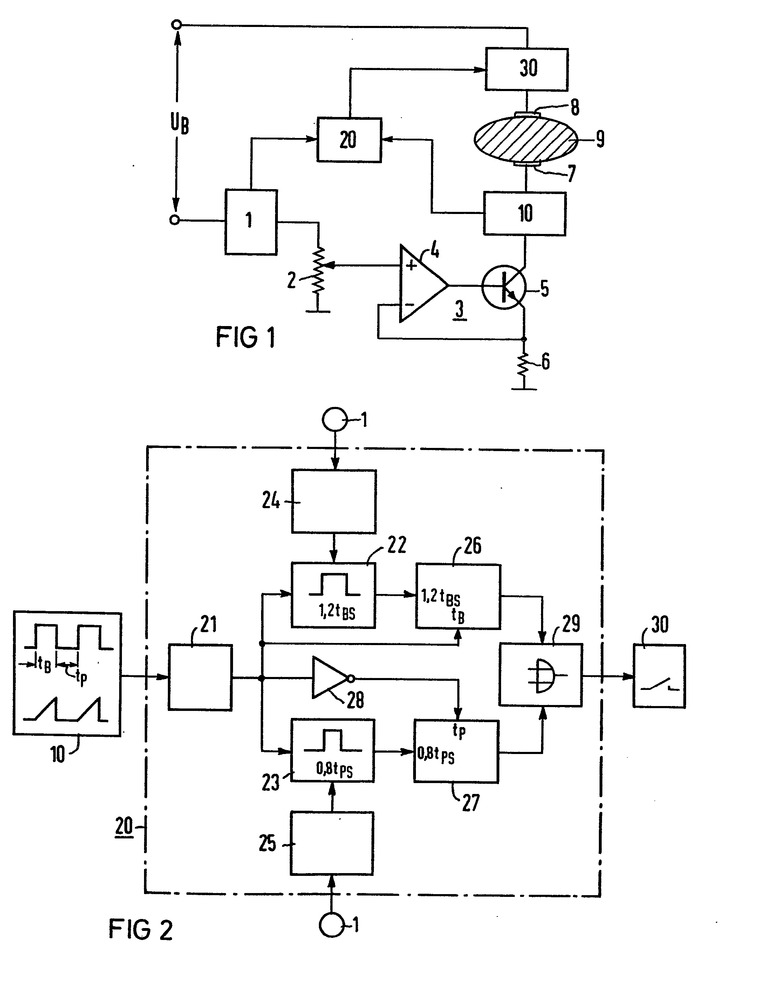

In der Fig. 1 ist mit $U_B$ eine Betriebsspannungsquelle bezeichnet. Der eine Zweig der Betriebsspannungsquelle liegt an einem Stromformgenerator 1 ; dieser liefert Gleichstromimpulse, beispielsweise Rechteckimpulse oder auch Dreieckimpulse mit einstellbaren Sollwerten für Impulsform, Impulsdauer und Impulsabstand und steht über ein Stromintensitätseinstellglied 2 (Potentiometer) mit einer Stromkonstantstufe 3 in Verbindung. In üblicher Weise ist die Stromformkontaktstufe 3 durch einen rückgekoppelten Operationsverstärker 4 mit nachfolgendem Transistor 5 und Emitter-Widerstand 6 gebildet. Bei Betrieb des Reizstromgerätes liegt der Kollektorausgang des Transistors 5 an einer der Stromzuführelektroden 7, während eine andere Stromzuführungselektrode 8 an die Betriebsspannungsquelle $U_B$ angeschlossen ist. Zwischen den Elektroden ist ein variabler Patientenwiderstand 9 angedeutet. Mit der Stromkonstantstufe 3 werden sich ändernde Patientenwiderstände so ausgeregelt, daß der Patientenstrom jeweils den am Stromformgenerator 1 vorgewählten Sollwerten folgt. Zwischen Stromkonstantstufe 3 und Stromzuführelektrode 7 ist eine Meßeinrichtung 10 eingeschaltet, mit der die tatsächlichen Istwerte der Zeitdauer der einzelnen Stromimpulse — $t_B$ — einerseits und des Impulsabstandes — $t_P$ — andererseits erfaßt werden. Von der Meßeinrichtung 10 wird eine Sicherheitsschaltung 20 zum Vergleich dieser Istwerte mit den am Stromformgenerator 1 eingestellten Sollwerten — $t_{BS}$, $t_{PS}$ — angesteuert, die gegebenenfalls eine Abschalteinrichtung 30 im Betriebsstromkreis aktiviert. Die Einheiten 10, 20 und 30 werden anhand der Fig. 2 im einzelnen beschrieben.

In der Fig. 2 ist die gesamte Sicherheitsschaltung 20 gestrichelt angedeutet ; sie umfaßt im einzelnen die Einheiten 21 bis 29 ; es sind 21 ein Schmitt-Trigger, 22 und 23 ein erstes und ein zweites monostabiles Zeitglied, 24 und 25 zugehörige Steuereinheiten, 26 und 27 ein erster und ein zweiter Komparator, 28 ein Negierungsglied sowie 29 ein Oder-Glied. 30 kennzeichnet die Einheit zur Abschaltung des Stromformgenerators 1 aus Fig. 1.

Die von der Meßeinrichtung 10 erfaßten Istwerte des impulsförmigen Patientenstromes — zwei Formen sind in Fig. 2 dargestellt — werden dem Eingang des Schmitt-Triggers 21 zugeführt und in eine Folge rechteckiger Istwertimpulse so umgeformt, daß die Länge der Istwertimpulse gleich ist der Dauer der einzelnen Impulse des Patientenstromes. Ebenso sind die Impulsabstände zwischen den Istwertimpulsen gleich den Abständen zwischen den Impulsen des Patientenstromes. Mit der Schaltung nach Fig. 2 werden diese Istwertimpulse mit einer Folge von Kontrollimpulsen verglichen, deren Dauer abhängig ist von den eingestellten Sollwerten für Impulsdauer — $t_{BS}$ — und für Impulsabstand — $t_{PS}$. Dabei ergibt sich im einzelnen folgender Funktionsablauf :

Die von dem Schmitt-Trigger 21 erzeugte Folge von Istwertimpulsen wird auf die beiden monostabilen Zeitglieder 22 und 23 gegeben, deren Rückkippzeiten über die Steuereinheiten 24 und 25 von den Sollwerten $t_{BS}$, $t_{PS}$ abhängen, z. B. auf 1,2 $t_{BS}$ bzw. 0,8 $t_{PS}$ eingestellt sind. Im einzelnen liefert also das erste retriggerbare Zeitglied 22 erste Kontrollimpulse, die jeweils mit der ansteigenden Flanke jedes Istwertimpulses beginnen und nach Ablauf der Rückkippzeit 1,2 $t_{BS}$ enden. Das zweite Zeitglied 23 liefert zweite Kontrollimpulse mit einer Länge von 0,8 $t_{BS}$, die jeweils mit der abfallenden Flanke jedes Istwertimpulses beginnen.

Die ersten Kontrollimpulse — von 22 — werden in dem Komparator 26 mit den Istwertimpulsen und die zweiten Kontrollimpulse — von 23 — mit Bezugsimpulsen verglichen. Die Bezugsimpulse werden durch das Negierungsglied 28 aus den Pausen zwischen den Istwertimpulsen erzeugt.

Von dem Komparator 26 wird dann ein Ausgangssignal abgegeben, wenn die Länge eines Istwertimpulses größer als die eines ersten Kontrollimpulses ist. Der zweite Komparator 27 liefert ein Ausgangssignal, wenn die Länge eines zweiten Kontrollimpulses größer als die eines Bezugsimpulses ist, der Abstand zweier Istwertimpulse also kleiner als der durch den zweiten Kontrollimpuls gegebene Mindestwert ist. Die Ausgangssignale der Komparatoren steuern über das ODER-Glied 29 das Abschaltglied 30 zur Abschaltung des Reizstromgerätes.

Die Abschaltung durch das Abschaltglied 30 kann durch Unterbrechen der Ausgangsleitung im Betriebsspannungszweig in Fig. 1 erfolgen. Es kann aber ebensogut auch die Ausgangsstufe 3 des elektromedizinischen Gerätes abgeschaltet werden.

### Patentansprüche

1. Reizstromgerät mit einem Betriebsstromkreis, in dem in Reihenschaltung eine Betriebsspannungsquelle ($U_B$), die Patientenelektroden (7, 8) und eine Meßeinrichtung (10) für den Istwert des Patientenstromes angeordnet sind, mit einem Stromformgenerator (1) mit Mitteln zum Einstellen des Sollwertes des Patientenstromes, mit einer Sicherheitsschaltung (20), der der Sollwert und der Istwert des Patientenstromes zugeführt wird und die eine Abschaltung des Reizstromgerätes durch eine Abschalteinrichtung (30) bewirkt, wenn der Patientenstrom bestimmte Grenzwerte überschreitet, dadurch gekennzeichnet,
daß der Stromformgenerator (1) eine Impulsfolge liefert und Mittel zur Einstellung der Sollwerte für die Impulsdauer ($t_{BS}$) und für den Impulsabstand ($t_{PS}$) aufweist,
daß Mittel (21) vorgesehen sind zur Umformung des impulsförmigen Patientenstromes in eine Folge von rechteckigen Istwertimpulsen, deren Dauer und Abstand gleich ist der Dauer und dem Abstand der Impulse des Patientenstromes,

daß zwei Komparatoren (26, 27) vorgesehen sind, mit deren Ausgangssignalen die Abschalteinrichtung (30) angesteuert wird,
daß der erste Komparator (26) die Dauer ($t_B$) der Istwertimpulse und die Dauer ($t_{BS}$) von ersten Kontrollimpulsen vergleicht und ein Ausgangssignal liefert, wenn die Dauer ($t_B$) eines Istwertimpulses größer als die Dauer ($t_{BS}$) eines ersten Kontrollimpulses ist,
daß ein erstes Zeitglied (22) vorgesehen ist, das durch die Anstiegsflanke jedes Istwertimpulses getriggert wird und die ersten Kontrollimpulse liefert, deren Länge von dem Sollwert ($t_{BS}$) für die Impulsdauer des Patientenstromes abhängig und größer als dieser ist,
daß dem zweiten Komparator (27) eingangsseitig zweite Kontrollimpulse und Bezugsimpulse zugeführt werden und dieser Komparator ein Ausgangssignal liefert, wenn die Dauer eines zweiten Kontrollimpulses größer als die eines Bezugsimpulses ist,
daß ein zweites, triggerbares Zeitglied (23) vorgesehen ist, das durch die Abstiegsflanke jedes Istwertimpulses gesetzt wird und das die zweiten Kontrollimpulse liefert, deren Länge von dem Sollwert ($t_{PS}$) für die Impulspause des Patientenstromes abhängig und kleiner als dieser ist,
wobei die Länge der Bezugsimpulse dem Abstand ·der Istwertimpulse und der Abstand der Bezugsimpulse der Breite der Istwertimpulse gleich ist.

2. Reizstromgerät nach Anspruch 1, dadurch gekennzeichnet, daß die Zeitglieder (22, 23) monostabile Multivibratoren sind.

### Claims

1. A current stimulator device with an operating circuit in which an operating voltage source ($U_B$), patient electrodes (7, 8) and a measuring device (10) for the actual value of the patient current are arranged in series,
with a current waveform generator (1) comprising means for adjusting the theoretical value of the patient current,
with a safety circuit (20) supplied with the theoretical value and the actual value of the patient current, and which disconnects the current stimulator device by means of a disconnecting device (30) when the patient current exceeds predetermined limit values, characterised in that :
the current waveform generator (1) supplies a pulse sequence and comprises means for adjusting the theoretical values for the pulse duration ($t_{BS}$) and for the pulse spacing ($t_{PS}$) ;
that means (21) are provided for converting the pulse-shaped patient current into an actual sequence of rectangular pulses whose duration and spacing is equal to the duration and spacing of the pulses of the patient current :
that two comparators (26, 27) are provided whose output signals serve to drive the disconnection device (30) ;
that the first comparator (26) compares the duration ($t_B$) of the actual value pulses and the duration ($t_{BS}$) of first control pulses and supplies an output signal if the duration ($t_B$) of an actual value pulse is greater than the duration ($t_{BS}$) of a first control pulse ;

that a first timer (22) triggered by the rising flank of each actual value pulse is provided and supplies the first control pulses whose length is dependent upon the theoretical value ($t_{BS}$) for the pulse duration of the patient current and is greater than the latter ;

that the second comparator (27) is supplied at its input with second control pulses and reference pulses and that this comparator supplies an output signal when the duration of a second control pulse is greater than that of a reference pulse ;

that a second triggerable timer (23) set by the falling flank of each actual value pulse is provided and supplies the second control pulses whose length is dependent upon the theoretical value ($t_{PS}$) for the pulse interval of the patient current and is smaller than the latter ;
where the length of the reference pulses is equal to the spacing between the actual value pulses and the spacing between the reference pulses is equal to the breadth of the actual value pulses.

2. A stimulator current device as claimed in Claim 1, characterised in that the timers (22, 23) are monostable multivibrators.

**Revendications**

1. Stimulateur avec un circuit de fonctionnement dans lequel sont montés en série une source de tension de fonctionnement ($U_B$), les électrodes (7, 8) pour le patient et un dispositif de mesure (10) pour la valeur instantanée du courant traversant le patient,

avec un générateur de conformation du courant (1) comportant des moyens pour régler la valeur de consigne pour le courant qui doit traverser le patient,

avec un circuit de sécurité (20) auquel sont appliquées la valeur de consigne et la valeur instantanée du courant devant traverser le patient, et qui provoque un débranchement du stimulateur à l'aide d'un dispositif de débranchement (30) lorsque le courant devant traverser le patient dépasse des valeurs limites déterminées, caractérisé par le fait,

que le générateur de conformation du courant (1) fournit un train d'impulsions et comporte des moyens pour régler les valeurs de consigne pour la durée des impulsions ($t_{BS}$) et pour l'intervalle entre impulsions ($t_{PS}$),

et que des moyens (21) sont prévus pour transformer le courant de forme impulsionnelle devant traverser le patient en une série d'impulsions rectangulaires de la valeur instantanée, dont les durées et les intervalles sont égaux à la durée et à l'intervalle des impulsions du courant devant traverser le patient,

que deux comparateurs (26, 27) sont prévus à l'aide des signaux de sortie desquels est attaqué le dispositif de débranchement (30),

que le premier comparateur (26) compare la durée ($t_B$) des impulsions de la valeur instantanée et la durée ($t_{BS}$) des premières impulsions de contrôle et fournit un signal de sortie lorsque la durée ($t_B$) d'une impulsion de valeur instantanée est supérieure à la durée ($t_{BS}$) d'une première impulsion de contrôle,

qu'il est prévu un premier dispositif de temporisation (22) qui est déclenché par le flanc croissant de chacune des impulsions de la valeur instantanée et qui fournit les premières impulsions de contrôle dont la longueur dépend de la valeur de consigne ($t_{BS}$) pour la durée d'impulsion du courant devant traverser le patient, et est supérieure à celle-ci,

et qu'on applique au second comparateur (27), du côté entrée, des secondes impulsions de contrôle et des impulsions de référence et ce comparateur fournit un signal de sortie lorsque la durée d'une seconde impulsion de contrôle est supérieure à celle d'une impulsion de référence,

qu'il est prévu un second dispositif de temporisation (23) qui est susceptible d'être déclenché, lequel circuit de temporisation est autorisé par le flanc croissant de chaque impulsion de la valeur instantanée et fournit les secondes impulsions de contrôle dont la longueur dépend de la valeur de consigne ($t_{PS}$) pour les intervalles entre impulsions du courant devant traverser le patient, est inférieure à celle-ci,

la réalisation étant telle que la longueur des impulsions de référence est égale à l'intervalle entre les impulsions de la valeur instantanée et l'intervalle entre les impulsions de référence est égale à la largeur des impulsions de la valeur instantanée.

2. Stimulateur selon la revendication 1, caractérisé par le fait que les dispositifs de temporisation (22, 23) sont des multivibrateurs monostables.

**FIG 1**

**FIG 2**